# EUROPEAN PATENT SPECIFICATION

(11) **EP 1 460 126 B1**
(45) Date of publication and mention of the grant of the patent: **17.06.2009**
(21) Application number: 04251501.5
(22) Date of filing: 17.03.2004
(51) Int. Cl.: C12M 1/26, B25J 19/06, G01N 1/22, C12Q 1/22

(54) **Microorganisms sampling method and microorganism sampling device**
Verfahren zur Entnahme von Mikroorganismen und Vorrichtung zur Entnahme von Mikroorganismen
Méthode de prélèvement de microorganismes et dispositif de prélèvement de microorganismes

(30) Priority: 20.03.2003 JP 2003078529; 30.04.2003 JP 2003125884
(43) Date of publication of application: 22.09.2004
(73) Proprietor: SHIBUYA KOGYO CO., LTD, Kanazawa-Shi Ishikawa-Ken (JP)
(72) Inventor: Yoshida, Shigeru, Kanazawa-Shi Ishikawa-Ken (JP); Yamamoto, Hisashi, Kanazawa-Shi Ishikawa-Ken (JP); Yoneda, Kenji, Kanazawa-Shi Ishikawa-Ken (JP); Oshima, Yasusuke, Kanazawa-Shi Ishikawa-Ken (JP); Kokubo, Mamoru, Kanazawa-Shi Ishikawa-Ken (JP); Akers, James E., Kansas City Missouri 64113 (US)
(74) Representative: Naylor, Matthew John

(56) References cited:
- WO-A-03/085126
- PATENT ABSTRACTS OF JAPAN vol. 2000, no. 13, 5 February 2001 (2001-02-05) & JP 2000 283910 A (DAIWA CAN CO LTD), 13 October 2000 (2000-10-13)
- PATENT ABSTRACTS OF JAPAN vol. 2000, no. 11, 3 January 2001 (2001-01-03) & JP 2000 219216 A (DAIWA CAN CO LTD), 8 August 2000 (2000-08-08)

## Description

### BACKGROUND OF THE INVENTION

### Field of the Invention

The present invention relates to a microorganism sampling method and a microorganism sampling device and specifically concerns a microorganism sampling method and a microorganism sampling device whereby microorganisms in a disinfected or sterilized working chamber are sampled into a sampling apparatus.

### Description of the Prior Art

Currently, an article processor is installed in a sterilized working chamber, sterilized articles are supplied to the working chamber, and the articles are processed under axenic conditions.

However, in reality, it is not possible to completely deny a probability that microorganisms are left due to insufficient sterilization of the working chamber; and bacteria and microorganisms enter the working chamber when the article processor is operated.

Hence, conventionally before and after an article processor is operated, an operator wears a clean suit and enters a working chamber, or the operator wears gloves and a half suit provided in the working chamber and samples bacteria and microorganisms in the working chamber from the outside of the working chamber. Culturing is performed to inspect the presence or absence of bacteria and microorganisms or the number of detected bacteria and microorganisms, and it is examined whether the axenic conditions in the working chamber are maintained when the article processor is operated.

Further, the following method of monitoring axenic conditions has been known: as to a sterilized working chamber composed of a sterile filling area for filling a container with beverage, sampling is performed on a conveyor lubricating material discharged from the working chamber, sterile water for cooling a container, beverage spilling from a container, and so on to inspect the presence or absence of bacteria and microorganisms in liquid, so that the axenic conditions in the working chamber is monitored (see Japanese Patent Laid-Open No. 2000-219216).

Further, the following device is known for sampling bacteria and microorganisms that float in a working chamber.

First as a device for sampling floating bacteria and microorganisms, a device is known which comprises a sampling apparatus for sampling bacteria and microorganisms, a holding member for holding the sampling apparatus, and suction means for sucking air in a working chamber by a motor (see Japanese Utility Model Laid-Open No. 58-84552, Japanese Utility Model Publication No. 6-13476, Japanese Patent Laid-Open No. 2000-304663).

Such a device is manually operated in a working chamber or set on a predetermined position in the working chamber. Air in the working chamber is sucked by the suction means, so that a current of air is produced around the sampling apparatus and causes the sampling apparatus to capture bacteria and microorganisms.

Moreover, a device is known which sucks air circulating in a working chamber to the outside of the working chamber, sprays water vapor onto the air to cause the water vapor to adhere to bacteria and microorganisms floating in the air, and detects the bacteria and microorganisms from the water vapor (see Japanese Patent Laid-Open No. 2000-283910).

However, even when an operator wears the clean suit, it is not possible to completely prevent the operator from bringing microorganisms into the working chamber. Further, when the operator wears the gloves and half suit for working, since there is a danger that a pin hole appears on the gloves and half suit and permits entry of microorganisms, incorrect positive may appear in the inspection results and the aseptic state may be degraded.

Meanwhile, in a technology described in Japanese Patent Laid-Open No. 2000-219216, microorganisms can be sampled without the necessity for an operator. However, when an article processor requiring no waste water is used, the configuration is not applicable. Besides, even when microorganisms are sampled from sampled liquid, it is not possible to know the position where the microorganisms are detected in a working chamber.

In the case where the devices of Japanese Utility Model Laid-Open No. 58-84552, Japanese Utility Model Publication No. 6-13476, Japanese Patent Laid-Open No. 2000-304663 are used in a disinfected or sterilized working chamber, air sucked by the suction means is exhausted into the working chamber as it is. Thus, a current of air may be disturbed in the working chamber and raise bacteria and microorganisms in the working chamber.

On the other hand, the device of Japanese Patent Laid-Open No. 2000-283910 does not raise bacteria or microorganisms in the working chamber but cannot specify where the bacteria and microorganisms have been sampled in the working chamber.

### SUMMARY OF THE INVENTION

In view of the above-described problems, the present invention provides a method and a device for sampling microorganisms according to claims 1 and 9 respectively. Accordingly, the present invention prevents microorganisms from entering a working chamber, samples microorganisms without any manual operations, prevents bacteria and microorganisms from rising in the working chamber, and readily specifies positions where bacteria and microorganisms have been sampled.

Since the handling means is provided in the working chamber, microorganisms are sampled without any manual operations, thereby improving the reliability of an inspection and preventing contamination in the working chamber.

The suction means is disposed outside the working chamber, so that air is not exhausted from the suction means into the working chamber and it is possible

Since the handling device is provided in the working chamber, microorganisms are sampled without any manual operations, thereby improving the reliability of an inspection and preventing contamination in the working chamber. Moreover, microorganisms can be sampled at predetermined intervals when the article processor is operated, the sampling having not been manually performed until now.

Air in the working chamber is sucked via the suction head during the operation of the article processor, so that it is possible to prevent a current of air from being disturbed around the article processor and prevent bacteria and microorganisms from adhering to articles and so on.

### BRIEF DESCRIPTION OF THE DRAWINGS

FIG. 1 is a plan view schematically showing a first isolator and a filling system of the present embodiment;
FIG. 2 is a sectional view showing a sampling apparatus;
FIG. 3 is a side view showing a wiping member;
FIG. 4 is a perspective view showing a housing rack;
FIGS. 5(a) and 5(b) show connecting means on the first isolator and a fourth isolator;

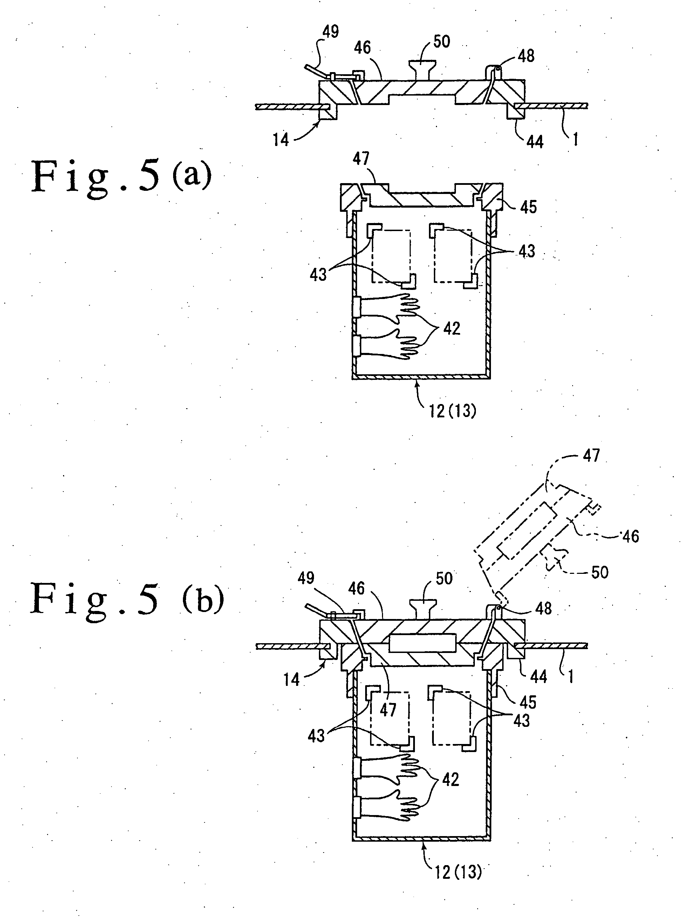

FIG. 5(a) shows a state before connection;
FIG. 5(b) shows a connected state;

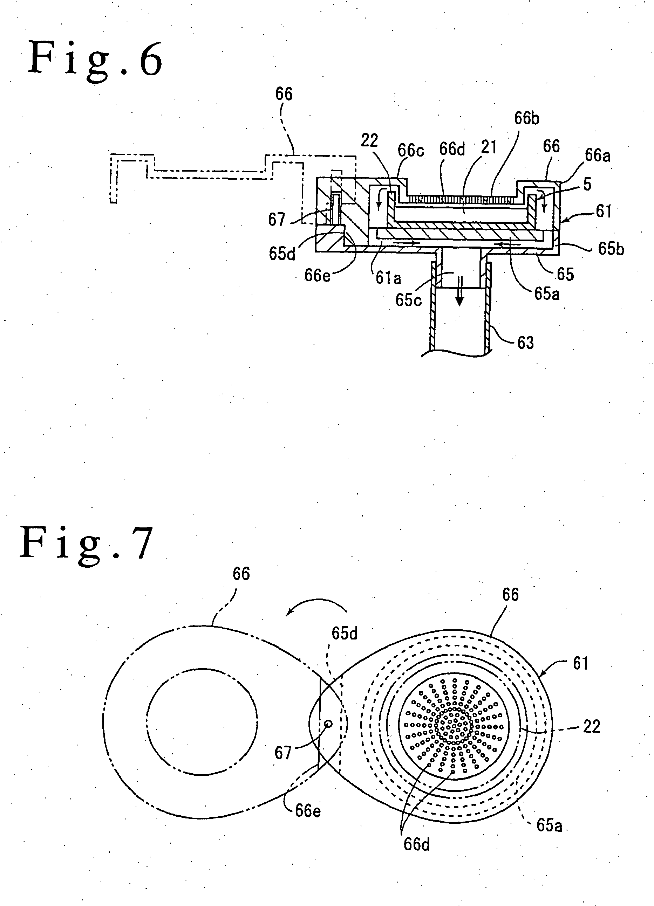

FIG. 6 is a sectional view showing a suction head;
FIG. 7 is a plan view showing the suction head;

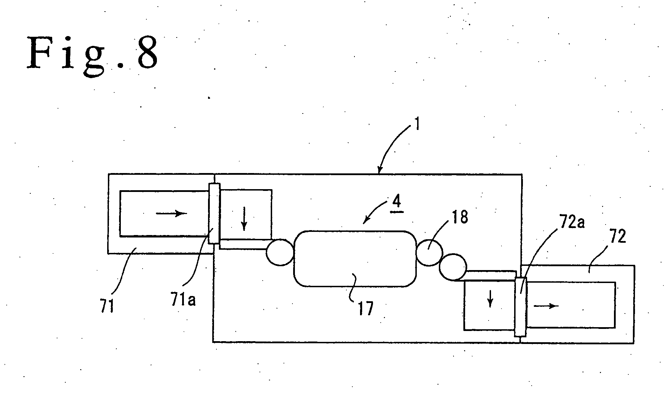

FIG. 8 is a plan view showing another embodiment;

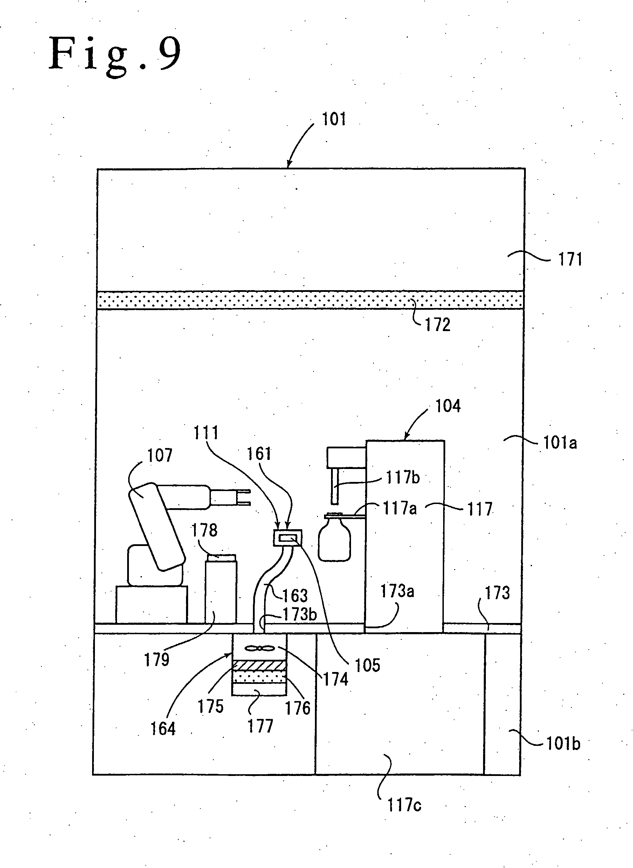

FIG. 9 is sectional view showing an isolator in another embodiment; and

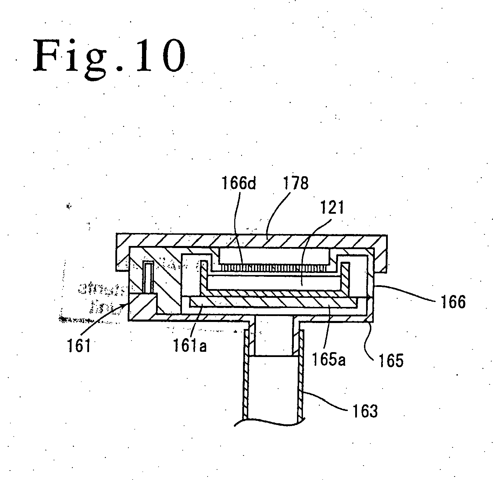

FIG. 10 is a sectional view showing a suction head of another embodiment.

### DETAILED DESCRIPTION OF THE EMBODIMENTS

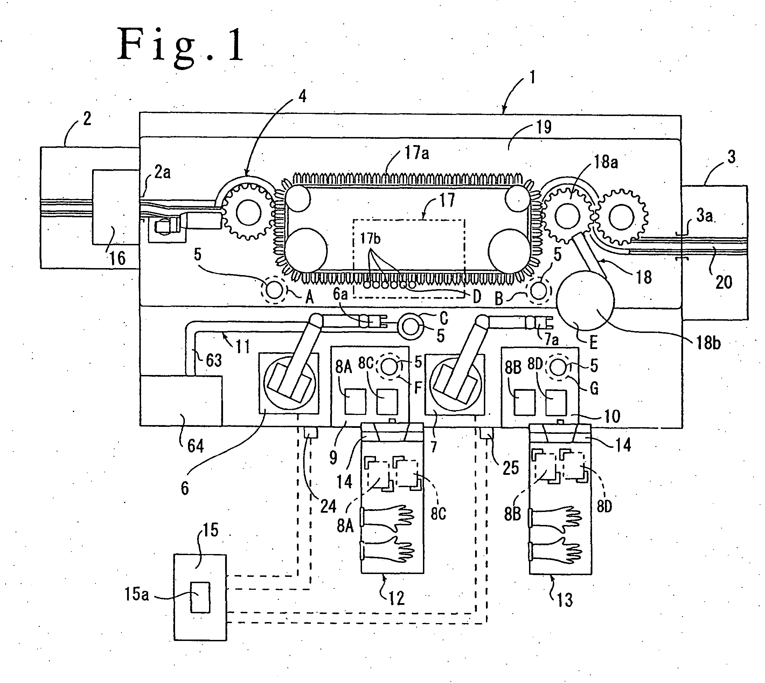

An illustrated example will be discussed below. Figure 1 shows that a first isolator 1 is provided as a main working chamber and a filling system 4 for chemicals is provided as an article processor inside second and third isolators 2 and 3, which are provided on both sides of the first isolator 1.

Further, in the first isolator 1, first and second robots 6 and 7 are provided as handling means which hold sampling apparatus 5 for sampling microorganisms, first and second placing tables 9 and 10 are provided for placing housing racks 8A to 8D for housing the plurality of sampling apparatus 5, and a floating bacteria sampling device 11 is provided for sampling floating microorganisms.

Moreover, below the illustrated first isolator 1, fourth and fifth isolators 12 and 13 serving as sub working chambers are provided so as to be separated by connecting means 14.

Then, the first and second robots 6 and 7 and so on are controlled by a control unit 15. The control unit 15 comprises recording means 15a for recording timing when sampling microorganisms.

The first isolator 1 isolates the filling system 4 from outside atmosphere and is kept at a predetermined positive pressure by air which is sterilized and supplied from a sterilized air supply device (not shown).

Then, the sterilized air supply device supplies sterilized air from above to below of the first isolator 1 in one direction so that dust does not rise in the first isolator 1.

Further, it is necessary to sterilize the inside of the first isolator 1 before the filling system 4 is operated. At this point, sterilization is performed by filling hydrogen peroxide vapor into the first isolator 1. The hydrogen peroxide vapor is supplied from a hydrogen peroxide sterilizer (not shown) which is connected to the first isolator 1.

Then, the first isolator 1 and the second and third isolators 2 and 3 are connected via openings 2a and 3a, and the inside of the second and third isolators 2 and 3 is set at a lower positive pressure than that of the first isolator 1.

Hence, the atmosphere in the first isolator 1 flows into the second and third isolators 2 and 3 through the openings 2a and 3a and the atmospheres in the second and third isolators 2 and 3 flow to the outside. Thus, it is possible to prevent microorganisms and so on from entering the first isolator 1, thereby maintaining the axenic condition of the first isolator 1.

Moreover, the filling system 4 comprises a container sterilizer 16 for sterilizing vial containers used as articles, a filling device 17 for filling chemicals into the vial containers, and a capping device 18 for capping the vial containers filled with chemicals. The filling device 17 and the capping device 18 are placed on a working table 19 provided in the first isolator 1.

The container sterilizer 16 is placed in the second isolator 2, sterilizes the vial containers supplied upstream of the container sterilizer 16, and supplies the sterilized vial containers to the first isolator 1.

Further, the filling device 17 comprises a transfer belt 17a for receiving the vial containers supplied from the container sterilizer 16, and a plurality of filling nozzles 17b for filling the vial containers with chemicals. The filling nozzles 17b can perform reciprocating motion so as to fill chemicals in accordance with the movement of the vial containers, which are transported by the transfer belt 17a.

Furthermore, the capping device 18 comprises a rotary capper 18a which receives vial containers one by one from the transfer belt 17a and caps the vial containers, and a cap feeder 18b for supplying caps to the capper 18a. Sterilized caps are prepared in the cap feeder 18b.

Then, the vial containers capped by the capper 18a are transported by the conveyer belt 20 to the third isolator 3 through the opening 3a.

Besides, only the upper surface of the working table 19 is exposed into the atmosphere of the first isolator 1. On the other hand, driving means (not shown) for driving the filling device 17 and the capping device 18 is provided on a lower part of the working table 19. Dust and the like generated from the driving means is prevented from entering the first isolator 1.

According to the above configuration, it is possible to supply sterilized air into the sterilized first isolator 1 and supply vial containers sterilized by the container sterilizer 16. Hence, it is possible to fill chemicals and perform capping under axenic conditions.

However, in reality, it can be considered that microorganisms are left in the first isolator 1. or microorganisms enter the first isolator 1 when the filling system 4 is operated. Hence, as with the case of the conventional technique, sampling of microorganisms is performed in the first isolator 1 to inspect the presence or absence of microorganisms in the present example.

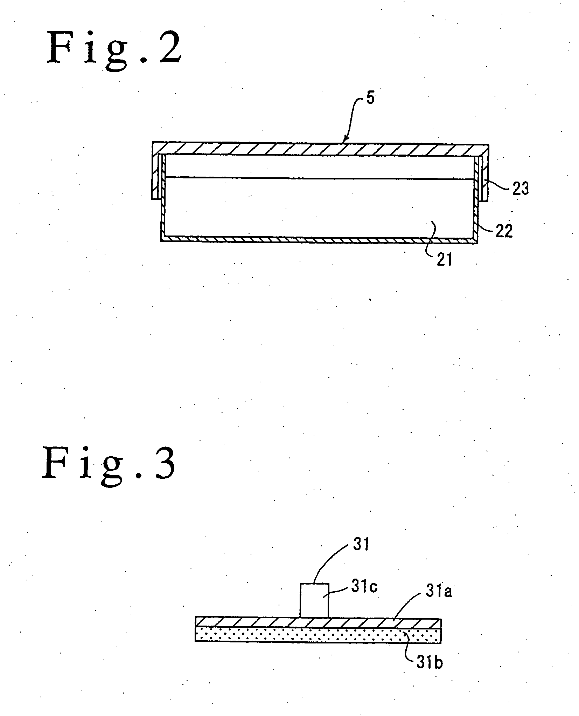

Then, as shown in Figure 2, the sampling apparatus 5 is composed of jellylike culture medium 21 for growing microorganisms, a petri dish 22 having the culture medium 21, and a lid 23 for closing the petri dish 22 from above. Different bar codes (not shown) are affixed to the lower surface of the petri dish 22.

The bar code is read by reading means 24 and 25 which are attached to the outer wall of the first isolator 1 via a glass window. When microorganisms are sampled by the sampling apparatus 5, the first and second robots 6 and 7 hold the sampling apparatus 5, the bar code is brought to the front of the reading means 24 and 25 to read the contents, and read data is outputted to the control unit 15.

Further, in the present example, microorganisms are sampled at the following first to fifth sampling positions A to E near the capping device 18 and the filling device 17 for filling the vial containers with chemicals.

First, the first and second sampling positions A and B are set on the upstream side and the downstream side of the filling nozzles 17b of the filling device 17, the lids 23 are removed from the sampling apparatus 5 and the petri dishes 22 are placed in an opening state in circles indicated by broken lines on the working table 19, and microorganisms falling naturally are sampled.

Next, the third sampling position C is set near the filling nozzles 17b. A suction head 61 of the floating bacteria sampling device 11 that will be described later is provided at the third sampling position C and the petri dish 22 is placed in the opening state, so that microorganisms floating near the filling nozzles 17b are sucked and sampled.

Moreover, the fourth sampling position D is set on a surface of predetermined one of the filling nozzles 17b. The surface of the filling nozzle 17b is wiped with a wiping member 31 described below, and the wiping member 31 having performed the wiping operation is stored in the sampling apparatus 5 placed at a first placing position F (in a circle indicated by a broken line) of the first placing table 9, so that microorganisms adhering to the surface of the filling nozzle 17b are sampled.

Furthermore, the fifth sampling position E is set at one predetermined portion of a part making contact with a cap in the cap feeder 18b. The fifth sampling position E is wiped with the wiping member 31, and the wiping member 31 having performed the wiping operation is stored in the sampling apparatus 5 placed at a second placing position G (a circle indicated by a broken line) on the second placing table 10, so that microorganisms adhering to the cap feeder 18b and caps are sampled.

Further, as shown in Figure 3, the wiping member 31 is constituted by a circular resin plate 31a, a nonwoven fabric 31b bonded on the lower surface of the resin plate 31a, and a handle 31c provided at the center of the upper surface of the resin plate 31a.

The resin plate 31a has a diameter storable by the petri dish 22. Ultra fine fibers are used for the nonwoven fabric 31b so as to sample microorganisms as many as possible.

Additionally, the handle 31c is shaped so as to be held by the first and second robots 6 and 7. When the microorganisms are sampled at the fourth and fifth sampling positions D and E, the handle 31c is held to wipe the sampling positions.

Besides, the wiping member 31 configured thus can be stored in the petri dish 22 and can be closed by the lid 23. The nonwoven fabric 31b is brought into contact with the culture medium 21, so that microorganisms adhering to the nonwoven fabric 31b are cultured.
In the present embodiment, the sampling apparatus 5 and the wiping member 31 are sealed into a packing bag in a sterilized state and then these are fed to the fourth and fifth isolators 12 and 13 while being sealed in the packing bag.

When the packing bags are opened after sterilization on the surface of the packing bag and the inner surfaces of the fourth and fifth isolators 12 and 13, it is possible to prevent microorganisms from adhering to the sampling apparatus 5 and the wiping member 31.

Additionally, the forms of the wiping member 31 and the sampling apparatus 5 for storing the wiping member 31 are not limited to the above. Any shapes and materials are acceptable as long as microorganisms can be effectively sampled by wiping off the sampling positions and these are stored and closed without being exposed to the outside. Moreover, as the culture medium 21 making contact with the nonwoven fabric 31b of the wiping member 31, it is desirable to use a liquid which can readily permeate the fabric.

Next, conventionally known industrial robots are used as the first and second robots 6 and 7, and holding means 6a and 7a are provided respectively on the ends of the robots.

When the holding means 6a and 7a hold the sampling apparatus 5, only the lids 23 are held under the control of the control unit 15, so that the lids 23 can be removed from the petri dishes 22. Further, the petri dishes 22 can be held and conveyed together with the lids 23.

Furthermore, parts of the first and second robots 6 and 7 are exposed into the first isolator 1 and are covered with a material resistant to corrosion caused by hydrogen peroxide. The surfaces of the first robots 6 and 7 can be sterilized while the inside of the first isolator 1 is sterilized by hydrogen peroxide vapor.

Subsequently, the housing racks 8A to 8D will be discussed. The housing racks 8A and 8B respectively store the plurality of sampling apparatus 5, and the housing racks 8C and 8D respectively store the plurality of wiping members 31.

Of the housing racks, the housing racks 8A and 8C are placed on the first mounting table 9 which is set near the forth isolator 12 in the first isolator 1, and the housing racks 8B and 8D are placed on the second mounting table 10 which is set near the fifth isolator 13.

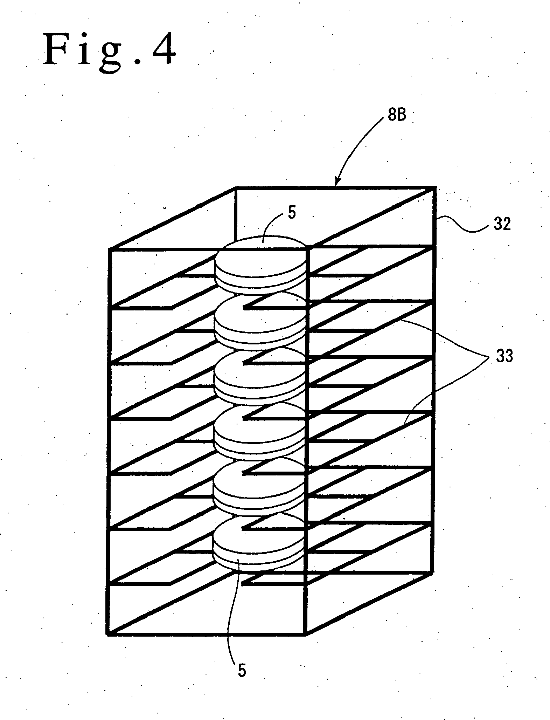

As shown in Figure 4, the housing racks 8A to 8D are each constituted by a stick metallic material which is resistant to corrosion caused by hydrogen peroxide. In an outer frame 32, placing parts 33 in a plurality of stages are provided for placing the sampling apparatus 5 and the wiping members 31 (Figure 4 shows the housing rack 8B).

A positioning member (not shown) for positioning the sampling apparatus 5 and the wiping member 31 is formed on each of the placing parts 33. Further, the placing parts 33 of the housing racks 8A and 8B are formed while the stick material is not partially provided, so that the holding means 6a and 7a can hold the petri dish 22 and the lid 23 from above and below to carry them in and out.

Moreover, the number of the stored sampling apparatus 5 and wiping members 31 can be arbitrarily changed by increasing or reducing the number of the placing parts 33. In the present example, the housing rack 8A stores nine sampling apparatus 5 and the housing rack 8B stores six sampling apparatus. The housing rack 8C stores six wiping members 31 and the housing rack 8D stores three wiping members 31.

Referring to Figures 5(a) and 5(b), the following will discuss the fourth and fifth isolators 12 and 13. Since the fourth and fifth isolators 12 and 13 have the same configuration, the fourth isolator 12 will be described below and the detailed description of the fifth isolator 13 will be omitted.

The fourth isolator 12 has its inside isolated from outside atmosphere and is configured so as to move. The fourth isolator 12 comprises the connecting means 14 for making connection with the first isolator 1, gloves 42 for permitting an operator to work in the fourth isolator 12, and positioning members 43 for positioning the housing racks 8A and 8C.

Further, as the connecting means 14, the first isolator 1 and the fourth isolator 12 respectively comprise connecting holes 44 and 45 permitting the passage of the housing racks 8A and 8C, and the connecting holes 44 and 45 respectively comprise partition walls 46 and 47 which can be opened and closed.

A hinge 48 for pivotally supporting the partition wall 46 is provided on the right side of the figures showing the inside of the first isolator 1 of the connecting hole 44, and a closing member 49 for closing the partition wall 46 is provided on the left side of the figures. The closing member 49 is rotated by the first robot 6, so that the closing state of the partition wall 46 is released.

Moreover, a handle 50 for opening and closing the partition wall 46 is provided at the center on the inside of the first isolator 1 of the partition wall 46. The handle 50 is held by the first robot 6, so that the partition wall 46 can be opened and closed.

Then, as shown in Figure 5(b), the connecting holes 44 and 45 are connected to each other. In this connecting state of the connecting holes 44 and 45, outside atmosphere does not flow into the first isolator 1 and the fourth isolator 12.

Further, the partition walls 46 and 47 are also connected to each other concurrently with the connection of the connecting holes 44 and 45, and the partition wall 47 is integrated with the partition wall 46 and is opened to the inside of the first isolator 1 while the hinge serves as the center.

Besides, according to the connecting means 14, of the connecting holes 44 and 45 and the partition walls 46 and 47, a part exposed outside before the first isolator 1 and the fourth isolator 12 are connected is not exposed into the first isolator 1 by making connection, resulting in no contamination on the first isolator 1.

Besides, since the configuration of the connecting means 14 has been conventionally known and is substantially equal to that of Japanese Patent Laid-Open No. 6-193323, the detailed description will be omitted.

Further, the following configuration is also applicable: the fourth and fifth isolators 12 and 13 are fixed on the first isolator 1 and are connected or separated via a partition wall which can be opened and closed. In this case, an opening/closing partition wall is further necessary for opening and closing the inside of the fourth and fifth isolators 12 and 13 to the outside. The configuration of the connecting means 14 can be simplified within a range separating the space of the first isolator 1 and the spaces of the fourth and fifth isolators 12 and 13.

The floating bacteria sampling device 11 is constituted of a suction head 61 which supports the sampling apparatus 5, an air intake passage 63 connected under the suction head 61, and suction means 64 for sucking an atmosphere in the first isolator 1 via the air intake passage 63 and the suction head 61.

First the suction head 61 will be described below. The suction head 61 is mounted near the filling nozzle 17b of the filling device 17 by using a stay (not shown). As shown in FIGS. 6 and 7, the suction head 61 comprises a placing part 65 for placing the petri dish 22 of the sampling apparatus 5, and a rectifying part 66 above the placing part 65. The air intake passage 63 is connected under the placing part 65.

The placing part 65 is somewhat larger in diameter than the petri dish 22. The placing part 65 comprises a placing member 65a which places the petri dish 22 on the upper surface and a cylindrical holding member 65b which holds the placing member 65a and forms a predetermined space between the holding member 65b and the placing member 65a. The lower surface of the holding member 65b comprises a connection port 65c for making a connection with the air intake passage 63.

Moreover, the rectifying part 66 is constituted of a cylindrical member 66a, which is polymerized on the upper surface of the outer periphery of the holding member 65b, a disk-shaped rectifying member 66b which is somewhat smaller in diameter than the petri dish 22 and is disposed a predetermined distance away from the surface of the culture medium 21, and a connecting member 66c which is shaped like a letter L in cross section, connects the cylindrical member 66a and the rectifying member 66b, and forms a small gap between the petri dish 22 and the connecting member 66c. An infinite number of through holes 66d are formed on the rectifying member 66b.

An air passage 61a is formed in the suction head 61 by the through holes 66d, a space formed between the rectifying part 66 and the petri dish 22, a space formed between the placing member 65a and the holding member 65b, and the connection portion 65c. Therefore, the internal space of the first isolator 1 is connected to the suction means 11 via the air passage 61a and the air intake passage 63.

Further, the cylindrical member 66a is provided horizontally with respect to the holding member 65b so as to be rotated by a rotating shaft 67, which is provided on the left of FIG. 6. Moreover, as shown in FIG. 7, uneven portions 65d and 66e are formed on the holding member 65b and the cylindrical member 66a. When the uneven portions 65d and 66e are engaged with each other, the holding member 65b and the cylindrical member 66a do not rotate from each other.

Hence, when the petri dish 22 on the placing part 65 is changed, it is necessary to move up the rectifying part 66 once to disengage the uneven portions 65d and 66e and then to rotate the rectifying part 66 about the rotating shaft 67.

Besides, although the outer surface of the suction device 64 is exposed into the first isolator 1, the inside of the device 64 is separated from the first isolator 1. A blower for suction and an exhaust duct (not shown) are provided in the suction device 64, and the sucked atmosphere in the first isolator 1 is discharged to the outside from the exhaust duct.

Additionally, Figure 1 schematically shows that the suction device 64 is away from the third sampling position C, it is desirable to minimize the length of the air intake duct 63 and place the air intake duct 63 near the third sampling position C.

Moreover, the rectifying part 66 can be attached and detached to the suction head 61. When the petri dish 22 is placed on the suction head 61, the first robot 6 may detach the rectifying part 66 and place the rectifying part 66 at an arbitrary position in the first isolator 1, and then, the petri dish 22 may be placed on the suction head 61.

With the above configuration, in the present example, sampling of bacteria and microorganisms is performed in the first isolator 1 in the following manner, and the presence or absence of bacteria and microorganisms is inspected in the first isolator 1.

First, the first isolator 1 and the fourth and fifth isolators 12 and 13 are not connected before the filling system 4 is operated, and the housing racks 8A to 8D are not supplied into the first isolator 1.

Then, in order to sterilize the first isolator 1, a hydrogen peroxide sterilizer is connected to the first isolator 1 and hydrogen peroxide vapor is supplied from the hydrogen peroxide sterilizer to entirely sterilize the inside of the first isolator 1.

In parallel with the sterilization, the partition wall 47 of the fourth isolator 12 is opened, the vacant housing racks 8A and 8C and a packing bag, which packs the plurality of previously sterilized sampling apparatus 5 and the wiping members 31 in a sterilizing state, are carried into the fourth isolator 12, the partition wall 47 is closed, and the packing bag and the housing racks 8B and 8D are similarly carried into the fifth isolator 13.

Then, the hydrogen peroxide sterilizer is also connected to these fourth and fifth isolators 12 and 13, and the inside of the fourth and fifth isolators 12 and 13 is sterilized, so that the outer surface of the packing bag and the housing racks 8A to 8D are sterilized.

When the sterilization is completed in the fourth and fifth isolators 12 and 13, an operator puts his/her hands into the gloves 42 to open the packing bag and stores the sampling apparatus 5 and the wiping members 31 one by one in the housing racks 8A to 8D, and the sampling apparatus 5 and the wiping members 31 are placed respectively at predetermined positions by the positioning member 43.

Then, when the fourth and fifth isolators 12 and 13 are connected to the first isolator 1 by using the connecting means 14, the operator transmits a signal for starting an operation to the control unit 15 and the following operation is automatically performed under the control of the control unit 15.

First, when the first robot 6 operates the closing member 49 and the handle 50 on the side of the fourth isolator 12 under the control of the control unit 15 to open the partition walls 46 and 47, holds the housing rack 8A and the housing rack 8C in the fourth isolator 12 in this order and places the housing racks at predetermined positions set on the first placing table 9. Thereafter, the handle 50 and the closing member 49 are operated again to close the partition walls 46 and 47.

At the same time, the second robot 7 is also activated. The second robot 7 similarly opens the partition walls 46 and 47 of the fifth isolator 13, transports the housing rack 8B and the housing rack 8D to the second placing table 10 from the fifth isolator 13, and closes the partition walls 46 and 47 again.

In the above-described state, the filling system 4 is not activated. In the present example, microorganisms are sampled once before the filling system 4 is activated.

The following will discuss the sampling of microorganisms at the first to fifth sampling positions A to E.

First, the following will discuss sampling of microorganisms at the first sampling position A. First, the first robot 6 takes out the sampling apparatus 5 from the housing rack 8A according to the predetermined operation, causes the reading means 24 to read the bar code of the petri dish 22, and places the sampling apparatus 5 at the first sampling position A.

Next, the first robot 6 removes the lid 23 from the petri dish 22 and stores the lid 23 once in the housing rack 8A. Since the culture medium 21 is exposed into the first isolator 1 by removing the lid 23, the petri dish 22 is set aside at the first sampling position A for a predetermined time period and samples microorganisms falling naturally to the first sampling position A.

When the predetermined time elapses, the first robot 6 takes out the lid 23 from the housing rack 8A, closes the petri dish 22, holds the sampling apparatus 5, and stores the sampling apparatus 5 in the housing rack 8A.

Further, according to the code number of the read bar code, the control unit 15 records in the recording means 15a that the sampling of this time is performed before the filling system 4 is activated (before production operation) and microorganisms are sampled at the first sampling position A, and according to the time for outputting an instruction of operating the first robot 6, the control unit 15 sequentially records timings between the time when the lid 23 is removed from the petri dish 22 and the time when the petri dish 22 is closed by the lid 23, as sampling timings of microorganisms.

At the second sampling position B, microorganisms are sampled by the second robot 7 performing an operation similar to the sampling of microorganisms at the first sampling position A by using the sampling apparatus 5 in the housing rack 8B.

As with the above case, according to the code number of the sampling apparatus 5 used for sampling microorganisms, the sampling position of this time and sampling timing are recorded in the recording means 15a.

The following will discuss the sampling of microorganisms at the third sampling position C. The first robot 6 firstly lifts up the rectifying part 66 of the suction head 61 and rotates the rectifying part 66 by 180° to open the placing part 65, so that the petri dish 22 can be placed on the placing part 65.

In this state, when the first robot 6 takes out the sampling apparatus 5 from the housing rack 8A and causes the reading means 24 to read the bar code of the petri dish 22, the sampling apparatus 5 is placed on the placing part 65, and the lid 23 is removed and is stored in the housing rack 8A.

Thereafter, the first robot 6 lifts up the rectifying part 66 and rotates the rectifying part 66 by 180° to close the placing part 65. Since the suction means 64 is activated to suck the atmosphere in the first isolator 1, microorganisms floating around the third sampling position C are sampled.

After a lapse of the predetermined time, when the first robot 6 opens the placing part 65 in the similar manner, the lid 23 is transported from the housing rack 8A, the petri dish 22 is closed, the sampling apparatus 5 is held and stored in the housing rack 8A. Then, the placing part 65 is closed by the rectifying part 66.

As with the case of the sampling of microorganisms at the first sampling position A and the second sampling position B, according to the code number of the sampling apparatus 5 used for sampling, the control unit 15 records the sampling position of this time and the sampling timing in the recording means 15a.

Further, the sampling of microorganisms at the fourth sampling position D will be described below. In this case, when the first robot 6 takes out the sampling apparatus 5 from the housing rack 8A and causes the reading means 24 to read the bar code of the petri dish 22, the first robot 6 transports the sampling apparatus 5 to the first placing position F on the first placing table 9, removes the lid 23, and stores the lid 23 in the housing rack 8A.

Next, the first robot 6 holds the handle 31c of the wiping means 31 stored in the housing rack 8C and moves the wiping means 31 while applying a predetermined pressure to the surface of the predetermined filling nozzle 17b, so that microorganisms adhering to the fourth sampling position D are wiped off.

Thereafter, the first robot 6 stores the wiping member 31 in the petri dish 22 at the first placing position F, closes the petri dish 22 by using the lid 23 stored in the housing rack 8A, holds the sampling apparatus 5, and stores the sampling apparatus 5 in the housing rack 8A.

Moreover, as with the sampling at the other sampling positions, according to the code number of the sampling apparatus 5 used for sampling microorganisms, the control unit 15 records the sampling position of this time and the sampling timing in the storage means 15a. Meanwhile, the control unit 15 records time when the first robot 6 actually starts the wiping operation in response to an operation instruction of the control unit 15.

At the fifth sampling position E, microorganisms are sampled by the second robot 7 performing an operation similar to the sampling of microorganisms at the fourth sampling position D by using the sampling apparatus 5 in the housing rack 8B and the wiping member 31 in the housing rack 8D. Microorganisms are sampled by wiping off the surface of the cap feeder 18b.

As with the case of the fourth sampling position D, the control unit 15 records the sampling position of this time and the sampling timing according to the code number of the sampling apparatus 5 used for sampling microorganisms.

As to the sampling operations performed by the first robot 6 at the first, third, and fourth sampling positions A, C, and D and the sampling operations performed by the second robot 7 at the second and fifth sampling positions B and E, shift to the subsequent sampling operation may be performed after completion of each sampling operation. However, the following control is more efficient:

Under the control of the first robot 6, when the sampling apparatus 5 is placed at the first sampling position A to start sampling microorganisms, the sampling apparatus 5 is placed also at the third sampling position C to start sampling microorganisms, and microorganisms are subsequently sampled at the fourth sampling position D.

Thereafter, after a lapse of predetermined time, the sampling apparatus 5 is sampled from the first sampling position A and the sampling apparatus 5 is sampled from the third sampling position C, thereby shortening time for sampling microorganisms at all the sampling positions.

As described above, when sampling of microorganisms at the sampling positions is completed before the filling system 4 is activated, the filling system 4 is activated to start filling of chemicals into vial containers. Thereafter, microorganisms are sampled at the first to fifth sampling positions A to E with predetermined timing when the filling system 4 is operated (during a production operation).

At this point, according to the code number of the bar code, the control unit 15 records that these sampling apparatus 5 sample microorganisms when the filling system 4 is operated.

Moreover, since the filling device 17 is operated at the fourth sampling position D, the filling nozzle 17b performs a reciprocating motion. The control unit 15 controls the first robot 6 in synchronization with the motion of the filling nozzles 17b, so that the filling nozzles 17b are wiped off.

In this way, since a danger is caused when microorganisms are sampled during the operation of the filling system 4, microorganisms have not been conventionally sampled manually. According to the present example, microorganisms can be sampled without the necessity for considering a danger.

Further, when it is assumed that the sampling operations performed by the first robot 6 at the first, third, and fourth sampling positions A, C, and D and the sampling operations performed by the second robot 7 at the second and fifth sampling positions B and E each constitute one cycle, in the present example, when sampling of three cycles is performed in total, the sampling apparatus 5 stored in the housing racks 8A and 8B and the wiping part members 31 stored in the housing racks 8C and 8D are exhausted.

Thus, in order to further sample microorganisms, it is necessary to replace the sampling apparatus 5 having sampled microorganisms during the operation of the filling system 4 with the sampling apparatus 5 not having sampled microorganisms and to add the wiping members 31 into the housing racks 8C and 8D.

Hence, the housing racks 8A and 8C for storing the sampling apparatus 5 having sampled microorganisms and the vacant housing racks 8B and 8D are transported to the fourth and fifth isolators 12 and 13 from the first isolator 1, in accordance with steps reversed from those of carrying in the housing racks.

Then, when the housing racks 8A to 8D are carried out from the first isolator 1, the sampling apparatus 5 having sampled microorganisms are taken out from the fourth and fifth isolators 12 and 13, another sampling apparatus 5 and wiping members 31 are stored in the housing racks 8A to 8D in the fourth and fifth isolators 12 and 13 in preparation for sampling of the subsequent cycle, and the housing racks 8A to 8D are carried into the first isolator 1 in accordance with steps similar to those of the first transportation.

With this operation, the cycle of sampling microorganisms can be repeated for an arbitrary number of times.

Next, when a planned production operation is completed on the filling system 4, microorganisms are sampled after the filling system 4 is operated (after the production operation), in a manner similar to the sampling before the operation.

At this point, according to the code number of the bar code, the control unit 15 records that these sampling apparatus 5 sample microorganisms after the filling system 4 is operated.

Then, when sampling of microorganisms after the operation of the filling system 4 is completed, the first and second robots 6 and 7 carry the housing racks 8A to 8D from the first and second placing tables 9 and 10 to the fourth and fifth isolators 12 and 13, in accordance with steps reversed from those of carrying in the housing racks 8A to 8D.

Thereafter, the sampling apparatus 5 which are taken out from the fourth and fifth isolators 12 and 13 after sampling of microorganisms are cultured for a predetermined time period to inspect the presence or absence of microorganisms.

If microorganisms are detected from the sampling apparatus 5, referring to the code number attached to the petri dish 22 and the data recorded by the recording means 15a makes it possible to know at which sampling positions the sampling apparatus 5 samples microorganisms and at which timings the sampling is performed before, during, or after the operation of the filling system 4.

Besides, the present example described the case where the housing racks 8A to 8D are carried in and out from the fourth and fifth isolators 12 and 13 to the first isolator 1 by the first and second robots 6 and 7. The housing racks 8A to 8D may be carried manually by using the gloves 42 provided in the fourth and fifth isolators 12 and 13.

In this case, positioning members are provided at predetermined positions on the first and second placing tables 9 and 10 and the housing racks 8A to 8D are placed thereon. Further, the sampling apparatus 5 after sampling are carried out from the first isolator 1 not only by the housing racks 8A to 8D but also by a conveyer which immediately carries out the sampling apparatus 5 after the sampling in a manner similar to the discharging operation of the vial containers.

Additionally, the above example described that the vial containers are successively supplied to the filling device 17 in the first isolator 1. However, as shown in Figure 8, sixth and seventh isolators 71 and 72 connected via shutters 71a and 72a may be provided instead of second and third isolators 2 and 3.

In this case, a predetermined number of vial containers having been sterilized and stored are transported to the first isolator 1 from the sixth isolator 71, and processed vial containers are transported to the seventh isolator 72 from the first isolator 1. When the shutters 71a and 71b are opened, the inside of the sixth and seventh isolators 71 and 72 is sterilized by hydrogen peroxide vapor so that the inside of the first isolator 1 is not contaminated.

In this configuration as well, microorganisms can be sampled just like the above example.

Next, the following will discuss another method for sampling microorganisms by using the wiping member 31 at the fourth and fifth sampling positions D and E.

First, the wiping members 31 are prepared in the housing racks 8C and 8D in the above example. It can be further considered that a wiping member formed like a belt is wrapped like a coil and is carried into the first isolator 1.

In this case, the wiping member is cut with a cutter and the like after being fed by a predetermined amount. When the first and second robots 6 and 7 use the wiping member, the end of the wiping member is held and cut.

Further, it can be also considered that the wiping member 31 is stored in the sampling apparatus 5 in advance, the lid 23 is removed to take out the wiping member 31 from the petri dish 22, and the wiping member 31 is stored again in the sampling apparatus 5 after sampling.

In any case, the first and second robots 6 and 7 can sample microorganisms at the fourth and fifth sampling positions D and E, and the wiping member can be stored in the sampling apparatus 5 as it is just like the above example.

Another embodiment of the floating bacteria sampling device will be described below. In the following explanation, the same members as those of the above embodiment will be indicated by reference numerals obtained by adding 100 to the original reference numerals.

FIG. 9 is a sectional view showing a first isolator 101 shaped like that of the above embodiment. In the present embodiment, the first isolator 101 is constituted of a working chamber 101a which has a chemical filling system 104 and a floating bacteria sampling device 111 therein and is kept under axenic conditions, an air chamber 171 which supplies air from the above of the working chamber 101a, and a machine room 101b which has the driving mechanism or the like of the chemical filling system 104 and the floating bacteria sampling device 111.

The air chamber 171 comprises a HEPA filter 172 between the working chamber 101a and the air chamber 171. Air supplied from the air chamber 171 is supplied from the above of the working chamber 101a.

Then, the air supplied by the air chamber 171 is exhausted from the below of the working chamber 101a and is circulated into the air chamber 171 again. Air in the working chamber 101a flows in one direction from the above to the below, so that dust or the like does not rise in the working chamber 101a.

Further, a sterilizing gas feeder (not shown) is connected to the working chamber 101a. The sterilizing gas feeder fills the working chamber 101a with sterilized gas such as hydrogen peroxide vapor, so that the inside of the working chamber 101a is sterilized.

Instead of a working table 19 of the above-described embodiment, a partition 173 is provided between the working chamber 101a and the machine room 101b to separate the atmospheres of the working chamber 101a and the machine room 101b, so that air in the machine room 101b does not flow into the working chamber 101a.

FIG. 9 shows a filling device 117 as the chemical filling system 104. In addition, the chemical filling system 104 comprises a container sterilizer for sterilizing a container and a capper for putting a lid onto a container in the working chamber 101a.

The filling device 117 comprises a transfer belt 117a for carrying a container and a filing nozzle 117b for filling a container with a liquid. A driving mechanism 117c such as a motor for driving the filling device 117 is mounted in the machine room 101b via a through hole 173a formed in the partition 173, so that dust generated by the driving mechanism 117c does not rise in the working chamber 101a.

Further, the driving mechanism (not shown) of first and second robots 106 and 107 is also mounted in the machine room 101b. On the other hand, first and second placing tables 9 and 10 are mounted on the upper part of the partition, and second to fifth isolators 2, 3, 12, and 13 are connected to the working chamber 101a.

The floating bacteria sampling device 111 comprises suction means 164 which is mounted in the machine room 101b and sucks air in the working chamber 101a and a suction head 161 which is connected to the suction means 164 via an air intake passage 163 and is mounted in the working chamber 101a. A sampling apparatus 105 for sampling bacteria is mounted in the suction head 161.

The suction means 164 is constituted of a suction blower 174 for sucking air, a catalyst 175 for removing the sterilized gas, a HEPA filter 176 for capturing bacteria in the air, and an exhaust duct 177 for exhausting air having been sucked by the suction blower 174 into the machine room 101b.

The air intake passage 163 is a pipelike member which is made of a material having resistance to corrosion by sterilized gas. The air intake passage 163 has one end connected to the suction blower 174 via the through hole 173b formed on the partition 173 and the other end connected to the suction head 161. Further, the air intake passage 163 is detachably attached to the suction means 164 and the suction head 161. When necessary, the air intake passage 163 can be washed separately.

As described above, the suction means 164 is mounted in the machine room 101b provided outside the working chamber 101a, so that air can be exhausted in the machine room 101b. Thus, it is possible to prevent bacteria from rising in the working chamber 101a or entering a container and prevent dust generated by the suction blower 174 of the suction means 164 from being released into the working chamber 101a.

Moreover, the suction head 161 is mounted near the filling nozzle 117b so as to sample bacteria floating around the filling nozzle 117b, thereby readily specify a position where bacteria have floated.

Further, the suction head 161 is mounted near the filling nozzle 117b of the filling device 117 by using a stay (not shown).

FIG. 10 is a sectional view showing the suction head 161. The suction head 161 of the present embodiment basically has a similar configuration except for an overcap 178 provided as a lid covering the upper part of a rectifying part 166.

The overcap 178 is handled by the second robot 107 and is placed on a placing stand 179 of FIG. 9 after being detached from the suction head 161.

According to the above configuration, the following will describe the steps of sampling bacteria and microorganisms that float in the working chamber 101a. The description will start from a state in which while the sampling apparatus 105 is not mounted in the suction head 161, the overcap 178 is mounted on the suction head 161.

In this state, the second robot 107 detaches the overcap 178 from the suction head 161 and places the overcap 178 onto the placing stand 179. Then, the second robot 107 holds the rectifying part 166 of the suction head 161, lifts the rectifying part 166 once, and rotates the rectifying part 166 by 180°.

Subsequently the second robot 107 holds the sampling apparatus 105 from a predetermined position in the working chamber 101a and places the sampling apparatus 105 onto a placing member 165a of a placing part 165. Thereafter, the second robot 107 returns the rectifying part 166 to the original position.

When the sampling apparatus 105 is mounted in the suction head 161, the suction means 164 is operated to suck bacteria floating in the working chamber 101a into the suction head 161. After the passage of through holes 166d, the bacteria are collided with a culture medium 121 and are captured thereon.

At this point, some bacteria may adhere to the inner surface of an air passage 161a or the air intake passage 163 without being captured in the culture medium 121 and such bacteria may reach the suction means 164.

However, bacteria having reached the suction means 164 are captured by the HEPA filter 176 and thus the bacteria are not released from the exhaust duct 177 into the machine room 101b. Further, as will be discussed later, bacteria having adhered to the air passage 161a and the air intake passage 163 are subjected to sterilization by sterilized gas in the working chamber 101a after completion of a filling operation.

When bacteria are sampled for a predetermined time, the suction means 164 is stopped. Thereafter, the second robot 107 detaches the sampling apparatus 105 from the suction head 161 in the opposite order of steps, holds the overcap 178 from the placing stand 179, and mounts the overcap 178 onto the suction head 161.

As described above, since the overcap 178 is mounted in the suction head 161, even when bacteria adhere to the air passage 161a and the air intake passage 163, the bacteria cannot enter the working chamber 101a, thereby preventing the bacteria from being released into the working chamber 101a.

The sampling apparatus 105 having captured bacteria is carried to the outside of the working chamber 101a while being closed. After a predetermined period of cultivation, the presence or absence of bacteria is detected.

In the present embodiment, bacteria are sampled by the floating bacteria sampling device 111 before, during, and after the operation of the filling device 117. The suction means 164 is operated particularly when the sampling apparatus 105 is set in the suction head 161 during the operation of the filling device 117.

Therefore, the suction of air in the working chamber 101a prevents a current of air from being disturbed around the filling nozzle 117b and prevents bacteria from entering a container as much as possible.

Furthermore, in the present embodiment, prior to the sampling of bacteria before the operation of the filling device 117 and subsequent to the sampling of bacteria after the operation of the filling device 117, the inside of the working chamber 101a is sterilized by the sterilizing gas feeder and simultaneously the floating bacteria sampling device 111 is sterilized.

At this point, the second robot 107 detaches the overcap 178 from the suction head 161 and places the overcap 178 onto the placing stand 179. In this state, sterilized gas is fed into the working chamber 101a from the sterilizing gas feeder.

When the sterilized gas is fed, the suction means 164 is operated to suck sterilized gas in the working chamber 101a to the suction means 164 via the suction head 161 and the air intake passage 163. At this moment, sterilization is performed on bacteria which have adhered to the inside of the air passage 161a and the air intake passage 163 without being captured by the sampling apparatus 105.

Further, since toxic sterilized gas having reached the suction means 164 is removed by the catalyst 175, the sterilized gas is not blown into the machine room 101b or is not fed to the outside, so that the driving mechanism 117c is not corroded.

After the elapse of a predetermined time, when the sterilizing gas feeder is stopped and sterilized gas is removed from the working chamber 101a after the elapse of another predetermined time, the sterilization in the working chamber 101a is completed and the suction of the suction means 164 is also stopped accordingly.

According to the configuration of the present embodiment, it is possible to prevent bacteria and microorganisms from rising in the working chamber, readily specify positions where bacteria and microorganisms have been sampled, and prevent a current of air from being disturbed in the working chamber when bacteria or microorganisms are not captured during the operation of the article processor.

Additionally, the vial containers are filled with chemicals on the filling system 4, 104 of each of the above examples. Instead of the vial containers, filling can be made in an ample, a bottle, a bag, and so on. In these cases, a melting and closing apparatus, a screw capper, and a welding apparatus serve as the capping device 18, and the filling devices 17, 117 can fill beverage and powder of chemicals and so on as well as chemicals.

In addition to the filling lines 4, 104, any device such as a processor for packing and wrapping articles under axenic conditions is applicable as long as the device can operate as an article processor under axenic conditions.

Moreover, the sampling position on the first isolators 1, 101 is not particularly limited. It is needless to say that the sampling position can be set at any position according to the characteristics of the article processor.

As described above, according to a method and a device for sampling microorganisms according to the present invention, handling means is provided in a working chamber, so that microorganisms are sampled without any manual operations, thereby improving the reliability of an inspection and preventing contamination in the working chamber.

Further, according to the microorganism sampling device of the present invention, since the handling means is used, microorganisms are sampled by using a plurality of sampling apparatus for a predetermined number of times when an article processor is operated, which has not been conventionally performed.

## Claims

1. A microorganism sampling method for sampling a bacterium and a microorganism in a disinfected or sterilized working chamber (1) into a previously closed sampling apparatus (5) supplied in the working chamber (1), the working chamber (1) having an article processor (4) therein,
**characterised in that** the method comprises the following steps:
providing a handling means (6,7) in the working chamber;
opening the previously closed sampling apparatus (5) with the handling means (6,7); and then
sampling a bacterium and a microorganism into the sampling apparatus (5); and
closing the sampling apparatus (5) with the handling means (6,7).

2. The microorganism sampling method according to claim 1, wherein the method further includes the steps of:
providing a suction means (64) disposed outside the working chamber (1) and a suction head (61) disposed at a required position in the working chamber (1);
placing the previously closed sampling apparatus (5) in the suction head (61); and after opening the previously closed sampling apparatus,
sucking air in the working chamber (1) during an operation of the article processor (4), via the suction head (61), to produce a current of air around the sampling apparatus (5) and thereby capturing said bacterium and a microorganism onto the sampling apparatus (5) from the air in the working chamber (1).

3. The microorganism sampling method according to claim 2, wherein the method further includes the steps of:
providing a detachable lid (66) on the suction head (61), the working chamber (1) and the suction means (64) being separated from each other when the lid (66) is mounted on the suction head (61); and
detaching the lid (66) while operating the suction means (64) to capture a bacterium and a microorganism and while changing the previously closed sampling apparatus (5) of the suction head (61) with a new one.

4. The microorganism sampling method according to claim 3, wherein the method further includes the step of detaching the lid (66) and operating the suction means (64) to suck air in the working chamber while disinfecting or sterilizing the working chamber (1).

5. The microorganism sampling method according to any one of claims 1 to 4, wherein the method further includes the steps of:
supplying a plurality of previously closed sampling apparatuses (5) in the working chamber (1); and
opening the previously closed sampling apparatuses (5) one by one with the handling means (6,7) at predetermined intervals in the working chamber (1) when operating the article processor (4).

6. The microorganism sampling method according to claim 5, wherein the method further includes the step of recording timings for opening and closing the previously closed sampling apparatuses (5) at predetermined intervals.

7. The microorganism sampling method according to any one of claims 1 to 5, wherein method further includes the following steps:
holding a sterilized wiping member (31) with the handling means (6,7);
wiping off the article processor (4) with the wiping member (31); and
storing the wiping member (31) in the previously closed sampling apparatus (5) so that a bacterium and a microorganism that have adhered to the article processor (4) are sampled.

8. The microorganism sampling method according to any one of claims 1 to 7, wherein the previously closed sampling apparatus (5) has a container (22) for storing a culture medium (21) and a lid (23) for closing the container (22), and the handling means (6, 7) opens the lid (23) to expose the culture medium (21) into the working chamber (1) and closes the lid (23) again after a lapse of predetermined time, so that a bacterium and a microorganism in the working chamber (1) are caused to adhere to the culture medium (21) and are sampled.

9. A microorganism sampling device comprising:
a disinfected or sterilized working chamber (1),the working chamber having an article processor (4) therein; **characterised in that** the microorganism sampling device has:
a handling device (6,7) which is disposed in the disinfected or sterilized working chamber (1), the handling device being arranged to handle a previously closed sampling apparatus (5) for sampling a bacterium and a microorganism; and
a control unit (15) for controlling the handling device;
wherein the control unit (15) is arranged to perform an operation of sampling a bacterium and a microorganism a predetermined number of times by using a plurality of said previously closed sampling apparatuses (5) when the article processor (4) is operated by activating the handling device, opening the previously closed sampling apparatus (5) supplied into the working chamber (1), and closing the sampling apparatus (5) again.

10. The microorganism sampling device according to claim 9, wherein the device further comprises
suction means (64) disposed outside the working chamber (1);
a suction head (61) disposed on a required position in the working chamber (1); and
an air intake passage (63) which connects the suction head (61) and the suction means (64),
wherein the device is capable of operating so that when the previously closed sampling apparatus (5) is opened, the previously closed sampling apparatus (5) is placed in the suction head (61) and air in the working chamber (1) is sucked by the suction means(64) , so that a current of air is produced around the sampling apparatus (5) and causes the sampling apparatus (5) to capture a bacterium and a microorganism from the air in the working chamber (1).

11. The microorganism sampling device according to claim 9 or claim 10, wherein the device further comprises a driving mechanism outside the working chamber (1), the driving mechanism being operable to drive the article processor (4).

12. The microorganism sampling device according to claims 10 or 11, wherein a catalyst for removing sterilized gas for sterilizing the working chamber (1) is attached to an exhaust port provided on the suction means for exhausting air in the working chamber (1) to an outside of the working chamber (1).

13. The microorganism sampling device according to claim 11 or 12, wherein the suction head (61) is disposed near an article processing position where the article processor (4) processes an article.

14. The microorganism sampling device according to any one of claims 9 to 13, further comprising recording means for recording timings for opening and closing the previously closed sampling apparatuses (5) at predetermined intervals.

15. The microorganism sampling device according to any one of claims 9 to 14, wherein the working chamber (1) comprises a main working chamber having the article processor (4) and the handling device (6,7) arranged therein and a sub working chamber (12, 13) connected to the main working chamber via a connecting hole capable of closing, and the previously closed sampling apparatus (5) before use is prepared in the sub working chamber (12, 13).

## Patentansprüche

1. Mikroorganismus-Probeentnahmeverfahren zur Entnahme eines Bakteriums und eines Mikroorganismus als Proben aus einer desinfizierten oder sterilisierten Arbeitskammer (1) in eine zuvor verschlossene Probeentnahmevorrichtung (5), die in der Arbeitskammer (1) bereitgestellt ist, wobei die Arbeitskammer (1) eine Gegenstandsbearbeitungsvorrichtung (4) aufweist,
**dadurch gekennzeichnet, dass** das Verfahren die folgenden Schritte umfasst:
die Bereitstellung eines Handhabungsmittels (6, 7) in der Arbeitskammer;
das Öffnen der zuvor verschlossenen Probeentnahmevorrichtung (5) mit dem Handhabungsmittel (6, 7); und die anschließende
Entnahme eines Bakteriums und eines Mikroorganismus als Proben in die Probeentnahmevorrichtung (5); und
das Verschließen der Probeentnahmevorrichtung (5) mit dem Handhabungsmittel (6, 7).

2. Mikroorganismus-Probeentnahmeverfahren nach Anspruch 1, worin das Verfahren ferner die folgenden Schritte umfasst:
die Bereitstellung eines Ansaugmittels (64), das außerhalb der Arbeitskammer (1) angeordnet ist, und eines Ansaugkopfes (61), der an einer erforderlichen Position in der Arbeitskammer (1) angeordnet ist;
das Platzieren der zuvor verschlossenen Probeentnahmevorrichtung (5) in dem Ansaugkopf (61); und das anschließende Öffnen der zuvor verschlossenen Probevorrichtung,
das Ansaugen von Luft in der Arbeitskammer (1) während eines Betriebs der Gegenstandsbearbeitungsvorrichtung (4) mithilfe des Ansaugkopfs (61), um einen Luftstrom um die Probeentnahmevorrichtung (5) zu erzeugen und dadurch das Bakterium und einen Mikroorganismus aus der Luft in der Arbeitskammer (1) auf der Probeentnahmevorrichtung (5) einzufangen.

3. Mikroorganismus-Probeentnahmeverfahren nach Anspruch 2, worin das Verfahren weiters folgende Schritte umfasst:
die Bereitstellung einer abnehmbaren Abdeckung (66) auf dem Ansaugkopf (61), wobei die Arbeitskammer (1) und das Ansaugmittel (64) voneinander getrennt sind, wenn die Abdeckung (66) auf dem Ansaugkopf (61) angebracht ist; und
das Abnehmen der Abdeckung (66) während des Betriebs des Ansaugmittels (64), um ein Bakterium und einen Mikroorganismus einzufangen und während die zuvor verschlossene Probeentnahmevorrichtung (5) des Ansaugkopfes (61) durch eine neue ausgetauscht wird.

4. Mikroorganismus-Probeentnahmeverfahren nach Anspruch 3, worin das Verfahren weiters den Schritt des Abnehmens der Abdeckung (66) und den Betrieb des Ansaugmittels (64) zum Ansaugen von Luft in der Arbeitskammer während der Desinfizierung oder Sterilisierung der Arbeitskammer (1) umfasst.

5. Mikroorganismus-Probeentnahmeverfahren nach einem der Ansprüche 1 bis 4, worin das Verfahren weiters folgende Schritte umfasst:
das Zuführen einer Vielzahl von zuvor verschlossenen Probeentnahmevorrichtungen (5) in der Arbeitskammer (1); und
das nacheinander erfolgende Öffnen der zuvor verschlossenen Probeentnahmevorrichtungen (5) mithilfe des Handhabungsmittels (6, 7) in vorbestimmten Zeitabständen in der Arbeitskammer (1) während des Betriebs der Gegenstandsbearbeitungsvorrichtung (4).

6. Mikroorganismus-Probeentnahmeverfahren nach Anspruch 5, worin das Verfahren weiters den Schritt des Aufzeichnens der Zeitpunkte des Öffnens und Verschließens der zuvor verschlossenen Probeentnahmevorrichtungen (5) in vorbestimmten Zeitabständen umfasst.

7. Mikroorganismus-Probeentnahmeverfahren nach einem der Ansprüche 1 bis 5, worin das Verfahren weiters die folgenden Schritte umfasst:
das Halten eines sterilisierten Wischelements (31) mit dem Handhabungsmittel (6, 7);
das Abwischen der Gegenstandsbearbeitungsvorrichtung (4) mit dem Wischelement (31); und
das Aufbewahren des Wischelements (31) in der zuvor verschlossenen Probeentnahmevorrichtung (5), sodass ein Bakterium und ein Mikroorganismus, die an der Gegenstandsbearbeitungsvorrichtung (4) haften geblieben sind, als Proben entnommen werden.

8. Mikroorganismus-Probeentnahmeverfahren nach einem der Ansprüche 1 bis 7, worin die zuvor verschlossene Probeentnahmevorrichtung (5) einen Behälter (22) zur Aufbewahrung eines Kulturmediums (21) und eine Abdeckung (23) zum Verschließen des Behälters (22) aufweist, und das Handhabungsmittel (6, 7) die Abdeckung (23) öffnet, um das Kulturmedium (21) in der Arbeitskammer (1) einer Einwirkung auszusetzen und die Abdeckung (23) nach Ablauf einer festgelegten Zeitdauer erneut verschließt, sodass ein Bakterium und ein Mikroorganismus in der Arbeitskammer (1) an dem Kulturmedium (21) haften bleiben und als Probe entnommen werden.

9. Mikroorganismus-Probeentnahmevorrichtung, umfassend:
eine desinfizierte oder sterilisierte Arbeitskammer (1), wobei die Arbeitskammer eine Gegenstandsbearbeitungsvorrichtung (4) aufweist;
**dadurch gekennzeichnet, dass** die Mikroorganismus-Probeentnahmevorrichtung Folgendes aufweist:
eine Handhabungsvorrichtung (6, 7), die in der desinfizierten oder sterilisierten Arbeitskammer (1) angeordnet ist, wobei die Handhabungsvorrichtung angeordnet ist, um eine zuvor verschlossene Probeentnahmevorrichtung (5) zur Entnahme eines Bakteriums und eines Mikroorganismus als Proben handzuhaben; und
eine Steuereinheit (15) zum Steuern der Handhabungsvorrichtung;
worin die Steuereinheit (15) angeordnet ist, um den Vorgang einer Entnahme eines Bakteriums und eines Mikroorganismus als Proben in festgelegter Häufigkeit unter Verwendung einer Vielzahl an zuvor verschlossenen Probeentnahmevorrichtungen (5) durchzuführen, wenn die Gegenstandsbearbeitungsvorrichtung (4) bedient wird, indem die Handhabungsvorrichtung aktiviert wird, die in der Arbeitskammer (1) bereitgestellte, zuvor verschlossene Probeentnahmevorrichtung (5) geöffnet wird und die Probeentnahmevorrichtung (5) erneut verschlossen wird.

10. Mikroorganismus-Probeentnahmevorrichtung nach Anspruch 9, worin die Vorrichtung weiters Folgendes umfasst:
ein Ansaugmittel (64), das außerhalb der Arbeitskammer (1) angeordnet ist;
einen Ansaugkopf (61), der an einer erforderlichen Position in der Arbeitskammer (1) angeordnet ist; und
einen Lufteinlassdurchlass (63), der den Ansaugkopf (61) mit dem Ansaugmittel (64) verbindet,
worin die Vorrichtung für einen Betrieb geeignet ist, dass wenn die zuvor verschlossene Probeentnahmevorrichtung (5) geöffnet wird, die zuvor verschlossene Probeentnahmevorrichtung (5) in den Ansaugkopf (61) platziert wird und Luft in der Arbeitskammer (1) durch das Ansaugmittel (64) angesaugt wird, sodass ein Luftstrom um die Probeentnahmevorrichtung (5) erzeugt wird und dazu führt, dass die Probeentnahmevorrichtung (5) ein Bakterium und einen Mikroorganismus aus der Luft in der Arbeitskammer (1) einfängt.

11. Mikroorganismus-Probeentnahmevorrichtung nach Anspruch 9 oder 10, worin die Vorrichtung weiters einen außerhalb der Arbeitskammer (1) vorliegenden Antriebsmechanismus umfasst, wobei der Antriebsmechanismus betreibbar ist, um die Gegenstandsbearbeitungsvorrichtung (4) anzutreiben.

12. Mikroorganismus-Probeentnahmevorrichtung nach Anspruch 10 oder 11, worin ein Katalysator zur Entfernung von sterilisiertem Gas zur Sterilisierung der Arbeitskammer (1) an eine auf dem Ansaugmittel bereitgestellte Auslassöffnung angebracht ist, um Luft aus der Arbeitskammer (1) nach außen abzulassen.

13. Mikroorganismus-Probeentnahmevorrichtung nach Anspruch 11 oder 12, worin der Ansaugkopf (61) nahe einer Gegenstandsbearbeitungsposition angeordnet ist, an welcher die Gegenstandsbearbeitungsvorrichtung (4) einen Gegenstand bearbeitet.

14. Mikroorganismus-Probeentnahmevorrichtung nach einem der Ansprüche 9 bis 13, weiters umfassend ein Aufzeichnungsmittel zum Aufzeichnen der Zeitpunkte des Öffnens und Verschließens der zuvor verschlossenen Probeentnahmevorrichtungen (5) in vorbestimmten Zeitabständen.

15. Mikroorganismus-Probeentnahmevorrichtung nach einem der Ansprüche 9 bis 14, worin die Arbeitskammer (1) eine Hauptarbeitskammer, in welcher die Gegenstandsbearbeitungsvorrichtung (4) und die Handhabungsvorrichtung (6, 7) angeordnet sind und eine Unterarbeitskammer (12, 13), die mit der Hauptarbeitskammer über ein verschließbares Verbindungsloch verbunden ist, umfasst und worin die zuvor verschlossene Probeentnahmevorrichtung (5) vor Gebrauch in der Unterarbeitskammer (12, 13) vorbereitet wird.

## Revendications

1. Procédé d'échantillonnage de micro-organisme pour échantillonner une bactérie et un micro-organisme dans une chambre de travail désinfectée ou stérilisée (1) dans un appareil d'échantillonnage préalablement fermé (5) fourni dans la chambre de travail (1), la chambre de travail (1) comportant un processeur d'article (4),
**caractérisé en ce que** le procédé comprend les étapes suivantes :
la fourniture d'un moyen de manipulation (6, 7) dans la chambre de travail ;
l'ouverture de l'appareil d'échantillonnage préalablement fermé (5) avec le moyen de manipulation (6, 7) ; puis
l'échantillonnage d'une bactérie et d'un micro-organisme dans l'appareil d'échantillonnage (5) ; et
la fermeture de l'appareil d'échantillonnage (5) avec le moyen de manipulation (6, 7).

2. Procédé d'échantillonnage de micro-organisme selon la revendication 1, dans lequel le procédé comprend en outre les étapes consistant à :
fournir un moyen d'aspiration (64) disposé à l'extérieur de la chambre de travail (1) et une tête d'aspiration (61) disposée à une position requise dans la chambre de travail (1) ;
placer l'appareil d'échantillonnage préalablement fermé (5) dans la tête d'aspiration (61) ; et après l'ouverture de l'appareil d'échantillonnage préalablement fermé,
aspirer l'air dans la chambre de travail (1) durant une opération du processeur d'article (4), par le biais de la tête d'aspiration (61), pour produire un courant d'air autour de l'appareil d'échantillonnage (5) et capturer ainsi ladite bactérie et un micro-organisme sur l'appareil d'échantillonnage (5) à partir de l'air dans la chambre de travail (1).

3. Procédé d'échantillonnage de micro-organisme selon la revendication 2, dans lequel le procédé comprend en outre les étapes consistant à :
placer un couvercle détachable (66) sur la tête d'aspiration (61), la chambre de travail (1) et le moyen d'aspiration (64) étant séparés l'un de l'autre lorsque le couvercle (66) est monté sur la tête d'aspiration (61) ; et
détacher le couvercle (66) tout en faisant fonctionner le moyen d'aspiration (64) pour capturer une bactérie et un micro-organisme et tout en remplaçant l'appareil d'échantillonnage préalablement fermé (5) de la tête d'aspiration (61) par un nouvel appareil.

4. Procédé d'échantillonnage de micro-organisme selon la revendication 3, dans lequel le procédé comprend en outre l'étape consistant à détacher le couvercle (66) et à utiliser le moyen d'aspiration (64) pour aspirer l'air dans la chambre de travail tout en désinfectant ou en stérilisant la chambre de travail (1).

5. Procédé d'échantillonnage de micro-organisme selon l'une quelconque des revendications 1 à 4, dans lequel le procédé comprend en outre les étapes consistant à :
fournir une pluralité d'appareils d'échantillonnage préalablement fermés (5) dans la chambre de travail (1) ; et
ouvrir les appareils d'échantillonnage préalablement fermés (5) un par un avec le moyen de manipulation (6, 7) à des intervalles prédéterminés dans la chambre de travail (1) lors du fonctionnement du processeur d'article (4).

6. Procédé d'échantillonnage de micro-organisme selon la revendication 5, dans lequel le procédé comprend en outre l'étape consistant à enregistrer des cadences d'ouverture et de fermeture des appareils d'échantillonnage préalablement fermés (5) à des intervalles prédéterminés.

7. Procédé d'échantillonnage de micro-organisme selon l'une quelconque des revendications 1 à 5, dans lequel le procédé comprend en outre les étapes suivantes :
le maintien d'un élément d'essuyage stérilisé (31) avec le moyen de manipulation (6, 7) ;
l'essuyage du processeur d'article (4) avec l'élément d'essuyage (31) ; et
le stockage de l'élément d'essuyage (31) dans l'appareil d'échantillonnage préalablement fermé (5) de sorte qu'une bactérie et un micro-organisme qui ont adhéré au processeur d'article (4) sont échantillonnés.

8. Procédé d'échantillonnage de micro-organisme selon l'une quelconque des revendications 1 à 7, dans lequel l'appareil d'échantillonnage préalablement fermé (5) comporte un contenant (22) pour stocker un milieu de culture (21) et un couvercle (23) pour fermer le contenant (22), et le moyen de manipulation (6, 7) ouvre le couvercle (23) pour exposer le milieu de culture (21) dans la chambre de travail (1) et ferme le couvercle (23) de nouveau après un laps de temps prédéterminé, de sorte qu'une bactérie et un micro-organisme dans la chambre de travail (1) sont amenés à adhérer au milieu de culture (21) et sont échantillonnés.

9. Dispositif d'échantillonnage de micro-organisme comprenant :
une chambre de travail désinfectée ou stérilisée (1), la chambre de travail comportant un processeur d'article (4) ; **caractérisé en ce que** le dispositif d'échantillonnage de micro-organisme comporte :
un dispositif de manipulation (6, 7) qui est disposé dans la chambre de travail désinfectée ou stérilisée (1), le dispositif de manipulation étant agencé pour manipuler un appareil d'échantillonnage préalablement fermé (5) pour échantillonner une bactérie et un micro-organisme ; et
une unité de commande (15) pour commander le dispositif de manipulation ;
dans lequel l'unité de commande (15) est agencée pour réaliser une opération d'échantillonnage d'une bactérie et d'un micro-organisme un nombre de fois prédéterminé en utilisant une pluralité desdits appareils d'échantillonnage préalablement fermés (5) lorsque le processeur d'article (4) est actionné en activant le dispositif de manipulation, en ouvrant l'appareil d'échantillonnage préalablement fermé (5) fourni dans la chambre de travail (1) et en fermant de nouveau l'appareil d'échantillonnage (5).

10. Dispositif d'échantillonnage de micro-organisme selon la revendication 9, dans lequel le dispositif comprend en outre :
un moyen d'aspiration (64) disposé à l'extérieur de la chambre de travail (1) ;
une tête d'aspiration (61) disposée à une position requise dans la chambre de travail (1) ; et
un passage d'entrée d'air (63) qui relie la tête d'aspiration (61) et le moyen d'aspiration (64),
dans lequel le dispositif est capable de fonctionner de sorte que, lorsque l'appareil d'échantillonnage préalablement fermé (5) est ouvert, l'appareil d'échantillonnage préalablement fermé (5) est placé dans la tête d'aspiration (61) et l'air dans la chambre de travail (1) est aspiré par le moyen d'aspiration (64), de sorte qu'un courant d'air est produit autour de l'appareil d'échantillonnage (5) et amène l'appareil d'échantillonnage (5) à capturer une bactérie et un micro-organisme à partir de l'air dans la chambre de travail (1).

11. Dispositif d'échantillonnage de micro-organisme selon la revendication 9 ou la revendication 10, dans lequel le dispositif comprend en outre un mécanisme d'entraînement à l'extérieur de la chambre de travail (1), le mécanisme d'entraînement pouvant être actionné pour entraîner le processeur d'article (4).

12. Dispositif d'échantillonnage de micro-organisme selon les revendications 10 ou 11, dans lequel un catalyseur pour éliminer le gaz stérilisé pour stériliser la chambre de travail (1) est attaché à un orifice d'échappement prévu sur le moyen d'aspiration pour évacuer l'air dans la chambre de travail (1) vers l'extérieur de la chambre de travail (1).

13. Dispositif d'échantillonnage de micro-organisme selon la revendication 11 ou 12, dans lequel la tête d'aspiration (61) est disposée à proximité d'une position de traitement d'article où le processeur d'article (4) traite un article.

14. Dispositif d'échantillonnage de micro-organisme selon l'une quelconque des revendications 9 à 13, comprenant en outre un moyen d'enregistrement pour enregistrer des cadences d'ouverture et de fermeture des appareils d'échantillonnage préalablement fermés (5) à des intervalles prédéterminés.

15. Dispositif d'échantillonnage de micro-organisme selon l'une quelconque des revendications 9 à 14, dans lequel la chambre de travail (1) comprend une chambre de travail principale ayant le processeur d'article (4) et le dispositif de manipulation (6, 7) agencés à l'intérieur et une sous-chambre de travail (12, 13) reliée à la chambre de travail principale par le biais d'un trou de raccordement capable de fermeture, et l'appareil d'échantillonnage préalablement fermé (5) avant utilisation est préparé dans la sous-chambre de travail (12, 13).
